(19) Europäisches Patentamt
European Patent Office
Office européen des brevets

(11) Publication number : **0 496 483 A2**

(12) # EUROPEAN PATENT APPLICATION

(21) Application number : **92300002.0**

(51) Int. Cl.$^5$ : **C12Q 1/68**

(22) Date of filing : **02.01.92**

(30) Priority : **22.01.91 US 644200**

(43) Date of publication of application :
**29.07.92 Bulletin 92/31**

(84) Designated Contracting States :
**AT BE CH DE DK ES FR GB GR IT LI LU MC NL PT SE**

(71) Applicant : **INTEGRATED DNA TECHNOLOGIES, INC.**
**1710 Commercial Park**
**Coralville, Iowa 52241 (US)**

(72) Inventor : **Walder, Joseph A.**
**2107 Slagle Circle**
**Iowa City, Iowa 52246 (US)**
Inventor : **Walder, Roxanne Y.**
**2107 Slagle Circle**
**Iowa City, Iowa 52246 (US)**

(74) Representative : **Thomson, Paul Anthony**
**Potts, Kerr & Co. 15, Hamilton Square**
**Birkenhead Merseyside L41 6BR (GB)**

(54) **Process to prevent contamination by amplified nucleic acid sequence.**

(57) An improved method for eliminating the problem of contamination by amplified nucleic acid sequences in which novel primers or polynucleotide substrates are used that can be cleaved from the product, thereby preventing it from being further amplified.

EP 0 496 483 A2

The present invention addresses the problem of cross-contamination by amplified nucleic acid sequences which arises in diagnostic and research related assays. More specifically, the invention relates to the use of novel primers for nucleic acid amplification reactions which can be cleaved from the amplified product. This interferes with further amplification of the product should there be any carried over into another sample.

A number of nucleic acid amplification methods have been devised to enable the detection of a low copy number of the target sequence. The most widely used of these methods currently is the polymerase chain reaction (Mullis, K.B. (1987) U.S. Patent No. 4,683,202). In the polymerase chain reaction (PCR) two oligonucleotide primers are used which circumscribe the nucleic acid sequence to be amplified and which hybridize to the complementary strands of that sequence. Extension of the primers with DNA polymerase copies the intervening sequence. With repeated cycles of denaturation of the newly synthesized double stranded DNA, reannealing of the primers and primer extension the target nucleic acid sequence is amplified exponentially. The number of copies of the original nucleic acid sequence produced is $2^n$, where n is the number of cycles of PCR. A further improvement in the process was achieved with the introduction of a thermostable DNA polymerase which allows extension of the primer (DNA synthesis) to be carried out at elevated temperatures, typically about 70°C (Erlich, E.A. et al, (1988) EPA 0258 017 A2). This enhances the specificity of the reaction and also has made it possible to automate PCR. The current cycle time with commercially available instruments is generally from 2 to 5 minutes.

PCR is a very powerful tool. Although the theoretical level of amplification ($2^n$) is often not achieved, it is possible to produce from a single molecule of the original target sequence $10^8$ to $10^9$ copies of the product, which can easily be detected by a variety of methods. This extreme sensitivity, however, gives rise to serious contamination problems which thus far have severely limited the use of PCR in clinical diagnostic assays and have posed difficulties in research applications of PCR as well. If the amplification reaction is carried out in a volume of 100 ul and $10^9$ copies are produced, then 1000 molecules of the product will be present in just 100 picoliters ($10^{-4}$ microliters) of the solution. Transfer of such small volumes, either by aerosol or mechanical means, to other samples is very difficult to avoid. This renders the assay system very susceptible to false positive results even with rigorous containment procedures. Other amplification methods in which large numbers of copies of the target are produced, such as transcription based amplification systems (see for example, Burg, L.J. et al. (1989) P.C.T. W089/01050; and Kwoh, D.Y. et al. (1989) Proceedings of the National Academy of Sciences USA 86, 1173-1177) and the ligase chain reaction (Barany, F. (1991) Proceedings of the National Academy of Sciences USA 88, 189-193) are subject to this same problem.

Two methods have been described to circumvent the carry-over problem associated with nucleic acid amplification reactions. In the first procedure deoxyuridine residues are incorporated into the primers used for PCR, or into the DNA synthesized by primer extension with deoxyuridine triphosphate (Longo, M.C. et al. (1990) Gene 93, 125-128). Prior to amplification, the sample is treated with the enzyme uracil DNA glycosylase to remove the base from uracil residues in any carry-over product which may be present. The product is then degraded by cleavage of the DNA strand at the abasic sites and thereby rendered incapable of further amplification. Native DNA in the sample is not affected by the treatment and the true target sequence, if present, can be amplified.

In a second method the sample is treated after amplification with an isopsoralen derivative which reacts with pyrimidine residues upon exposure to UV light (Cimino, G.D. and Isaacs, S.T. The Fifth San Diego Conference on Nucleic Acids: New Frontiers, November 14-16, 1990, p. 17). Nucleic acids modified in this manner retain their ability to hybridize but cannot be copied. If such a product were transferred to another sample it would not be amplified.

Both of these approaches suffer disadvantages. In the uracil glycosylase method an extra enzymatic step is required. If deoxyuridine is incorporated into the primers they must be added following the uracil glycosylase treatment since they too would be substrates for the enzyme. This introduces another step at which carry-over may occur and makes it impossible to control for contamination within the primers themselves. If deoxyuridine is incorporated into the newly synthesized DNA, the product would not be detectable by hybridization after treatment with the enzyme. The process therefore cannot be carried out immediately post-amplification, but only after detection of the product necessitating further manipulation of the sample with attendant risks of cross-contamination. The isopsoralen method requires the use of costly and carcinogenic reagents. A uniform set of reaction conditions cannot be applied to all target sequences. The concentration of isopsoralen and level of light exposure required is dependent on the length and base composition of the product. The method is not highly effective with shorter sequences. Moreover, the procedure cannot be carried out prior to amplification because the native DNA within the sample would also be modified.

The above methods rely on the incorporation of a modified base into the amplified product which either directly prevents replication or allows the product to be degraded. The present invention departs from this approach.

In this invention, the carry-over problem is eliminated by employing novel RNA containing primers for the amplification reaction which can be cleaved from the product. Cleavage of the primers at the ribonucleotide linkage interferes with further amplification. Since neither the original target sequence nor the extension product are modified, the procedure can be carried out both pre- and post-amplification or at both steps. In the preferred method, a single ribose residue is incorporated at the 3′-terminus of the primer. Since such a primer is not itself subject to degradation it can be added to the sample prior to the cleavage reaction. The method can also be used with amplification procedures in which the nucleic acid substrate is directly incorporated into the product such as the ligase chain reaction.

Accordingly, the primary objective of the present invention is to provide an improved method for eliminating the carry-over problem associated with nucleic acid amplification systems based on the use of primers or polynucleotide substrates for the reaction which can be cleaved from the amplified product wherein the cleavable linkage is a ribonucleotide residue.

Yet a further objective of the invention is to provide RNA containing primers and polynucleotide substrates which are useful in nucleic acid amplification reactions.

Still a further objective of the present invention is to provide methods for the cleavage of an RNA containing primer from an amplified nucleic acid sequence.

Another further objective of the invention is to provide a method for isolating an amplified nucleic acid sequence in which the product is first bound to a solid support and then released by cleavage of the primer.

## Brief Description of the Drawings

Fig. 1 illustrates the use of RNA containing primers in accord with the present invention to eliminate the problem of contamination by amplified nucleic acid sequences in PCR. R represents a ribose residue at the 3′-terminus of the primers.

Fig. 2 illustrates the use of primers containing a ribose residue to protect against contamination in transcription based amplification systems. R represents a ribose residue. Pr is a promoter sequence.

Fig. 3 illustrates the use of polynucleotide substrates in the ligase chain reaction which contain ribonucleotide residues to eliminate contamination by amplified nucleic acid sequences. R represents a ribose residue.

Fig. 4 represents a photograph of an ethidium bromide-stained agarose gel of PCR products obtained after amplification of a 130 base pair segment of human cytomegalovirus (CMV) DNA. The substrate for the reaction was either the 130-mer prepared with PCR primers incorporating a ribose residue (lanes 2 and 3) or native CMV DNA (lanes 4 and 5). Pretreatment with base which cleaves the PCR primers from the 130-mer prevents further amplification of the product (lane 3) but did not interfere with replication of the CMV DNA target sequence (lane 5).

## Summary of the Invention

An improved method for eliminating the problem of contamination of amplified nucleic acid sequences wherein novel primers containing ribonucleotide residues are employed which can be cleaved from the product, thereby preventing the product from being further amplified. Both chemical and enzymatic methods for cleavage are provided. The process can be employed both pre- and post-amplification or at both steps. The method can be utilized in conjunction with many different amplification systems including, but not limited to, PCR, transcription based amplification reactions and the ligase chain reaction. The method can also be used for the isolation of an amplified nucleic acid sequence wherein the product is first bound to a solid support and then liberated by cleavage of the primer.

## Detailed Description of the Invention

In nucleic acid amplification procedures one or more primers or polynucleotide substrates are employed which first hybridize to the target sequence and become incorporated into the product. Because a large number of copies of the product are made, such reactions are subject to contamination problems due to carry-over of the amplified product to other samples. In the present invention this problem is eliminated by cleaving the primers from the amplified product. The invention resides in the use of novel primers containing ribose residues for amplification reactions which can be excised from the product under conditions that do not affect DNA. It can be utilized in conjunction with any primer dependent amplification process, including but not limited to PCR and transcription based amplification systems, and methods such as the ligase chain reaction in which a polynucleotide substrate is directly incorporated into the amplified product.

The use of the present invention with PCR is illustrated in Fig. 1. The PCR product (I) is a double stranded

DNA molecule bounded by the two primers used in the reaction. The primers are generally 15 to 30 nucleotides in length. The DNA synthesized by primer extension may be up to several thousand nucleotides. If copies of the product are transferred to another sample they would be amplified exponentially giving rise to a false positive result. With the invention detailed herein, this problem is avoided by cleaving the primers from the amplified product. The cleavable linkage within the primers is a ribose residue (R). Such a linkage may be introduced at a single site or at multiple sites within the primers. It is desirable that the RNA residues lie within the first 5 nucleotides from the 3′-end so that most or all of the primer sequence can be cleaved from the product. A primer having a single ribose residue at the 3′-terminus is preferred. This allows the entire primer to be excised. Moreover, since the ribose residue is at the very end of the primer, the primer itself is not subject to degradation and can be added to the sample before the cleavage reaction. Such primers can be utilized as substrates by DNA polymerases including, but not limited to , E.coli DNA polymerase I, Thermus aquaticus (Taq) DNA polymerase and reverse transcriptase. The RMA residue once incorporated into the product can also be copied by these enzymes. As shown in Example 1, a primer having a 3′-terminal ribose residue can be used in the PCR with Taq DNA polymerase as efficiently as an all DNA primer without any change in the reaction protocol.

The DNA fragments formed after cleavage of the primers, (A′) and (B′), can no longer be amplified exponentially because the products made in subsequent rounds of synthesis cannot serve a: future templates. The newly synthesized bottom strand (C) in reaction 2 cannot serve as a template because it lacks a binding site for primer 1. The newly synthesized top strand (D) in reaction 3 cannot serve as a template because it lacks a binding site for primer 2. Linear amplification of fragments A′ and B′ can occur, but this does not give rise to a significant level of background compared to exponential amplification of the true target. For example, if $10^4$ molecules of the PCR product (I) were accidentally transferred to another sample and cleaved to give $10^4$ molecules of fragments A′ and B′, $2.5x10^5$ copies of C and D would be formed after 25 cycles of PCR. This is 100-fold lower than the number of copies of the product (approximately $3x.10^7$) that would be produced by exponential amplification from only a single molecule of the native target sequence, if present in the sample. Even if $2.5x10^5$ molecules gave rise to a measurable signal in the final detection assay, it would be evident that this was due to contamination since it would be 100-fold less than the signal obtained starting with only one molecule of the true target. Although generally not required, a further reduction in carry-over background can be achieved utilizing an assay format which would distinguish (C) and (D) from the actual PCR product (I). For example, since (C) lacks the 3′-terminal sequence of the bottom strand of (I), the latter could be selectively isolated by hybridization to a solid support and quantitated using a sandwich assay.

Since the DNA synthesized by primer extension is not modified nor affected by the reactions used to cleave the primers (see below), the process can be carried out immediately following PCR, before detection of the product. Because the sample DNA is not affected, the process can also be performed prior to amplification. To reduce the risk of carry-over even further the method may be applied at both pre- and post-amplification steps.

In transcription based amplification reactions three products are formed: two complementary DNA fragments, shown as a duplex in Fig. 2, and an RNA transcript of that sequence. One or both of the oligonucleotide primers incorporated into the DNA product includes a promoter, an RNA polymerase binding site, to initiate transcription. Multiple copies of RNA are produced by transcription of each DNA template. In turn, each molecule of DNA serves as a template for the synthesis of a complementary DNA (cDNA) copy by reverse transcriptase. In the example in Fig. 2, primer 2 serves to initiate the synthesis of the first strand of the cDNA. Synthesis of the second strand is initiated by primer 1 which contains the promoter sequence. Cleavage of the primers from the two DNA strands interferes with further amplification of these products. Removal of primer 1 from the top strand yields a dead-end product (E′) incapable of further amplification. (E′) could be converted to a double stranded DNA molecule by extension of primer 2, but this fragment would lack a promoter and, therefore, would not support transcription. After cleavage of primer 2 from the bottom strand the product (F′) could be converted to a double stranded molecule by hybridization and extension of primer 1. Although this species could be transcribed, a truncated transcript would be produced lacking a binding site for primer 2. Hence the RNA synthesized could not be converted to a cDNA copy and consequently the level of amplification would be negligible. To fully circumvent the carry-over problem, it is also necessary to prevent the RNA product from being further amplified. In this regard use of a ribose residue as the cleavable linkage within the primer has a unique advantage: the RNA product can be degraded in the same reaction used to remove the primers.

The ligase chain reaction is illustrated in Fig. 3. Two or more polynucleotide substrates are joined to form the product in a template dependent reaction catalyzed by DNA ligase. To enhance the stringency of the reaction a thermophilic DNA ligase is employed. Incorporation of ribose residues into the nucleic acid substrates allows the product to be destroyed, thereby eliminating the risk of cross-contamination. The ribose residue should lie within 5 nucleotides of the point of ligation to ensure that the cleavage product cannot subsequently serve as a template.

The ribonucleotide linkage of primers and polynucleotide substrates useful in the invention can be selectively cleaved by both enzymatic and chemical methods. Enzyme activities useful for this purpose include but are not limited to RNaseIII, which will cleave the ribose linkage of the primer if present in an RNA:RNA duplex., RNaseH, which cleaves the RNA strand of an RNA:DNA duplex and single stranded ribonucleases. If E.coli RNaseH is utilized to excise the primer a stretch of 4 ribose residues is required for efficient cleavage (Hogrefe, H.H. et al. (1990) Journal of Biological Chemistry 265 , 5561-5566). Recently the major human RNaseH activity present in K562 erythroleukemia cells has been purified (Eder, P.S. and Walder, J.A. (1991) Journal of Biological Chemistry, in press). This enzyme, designated human RNaseHI, will cleave the RNA containing strand of a DNA-RNA-DNA:DNA duplex with only a single ribose residue. For cleavage of the primer using a single stranded ribonuclease, the amplified product must first be denatured, for example, by heating, to separate the two strands. Of the enzymatic methods, the use of a single stranded ribonuclease is preferred. Many such enzymes are available which have been well characterized, are inexpensive and very stable, examples of which include ribonuclease A which cuts after U and C residues, ribonuclease $T_1$, which cuts after G, and ribonuclease $T_2$ which cuts after A. Moreover, all copies of the product, which may initially exist in multiple forms, are converted to a single stranded species by denaturation of the duplex which can then be cleaved by the enzyme.

RNA containing primers can also be cleaved from the amplified product by alkaline hydrolysis. This procedure is simpler, more robust and less costly than the enzymatic methods, and is, therefore, most preferred. Typical reaction conditions for base catalyzed hydrolysis of the ribose linkage are 0.6 N NaOH for 30 minutes at 90°C (see Example 2).

Primers containing ribose residues can be readily prepared with current, automated methods of oligonucleotide synthesis utilizing either the phosphoramidite or hydrogen phosphonate coupling procedures. The synthesis proceeds from the 3′ to 5′ direction with the 3′-terminal residue attached to a solid support, most commonly controlled-pore glass. Solid supports with each of the four ribose residues bound are commercially available. The attachment is through an ester linkage to either the 2′ or 3′-hydroxyl group; the adjacent OH-group not linked to the support is blocked with an acetyl group. For the synthesis of primers having only a 3′-terminal ribose residue, the subsequent addition of deoxynucleotide monomers, removal of the protecting groups and purification of the product are carried out in exactly the same manner as for the synthesis of an all DNA fragment. If the ribose residue is recessed from the 3′-end, a 2′-OH protecting group is required which will remain intact during the $NH_4OH$ treatment used to deblock the bases. The t-butyldimethylsilyl protecting group is currently preferred and the corresponding phosphoramidite monomers for each of the four ribose residues are commercially available. Polynucleotides up to about 100 residues can be readily synthesized chemically. Longer sequences containing ribose residues can be prepared using a combination of chemical and enzymatic methods in which shorter fragments are joined using DNA or RNA ligase.

The present invention also provides a convenient method to isolate one or both strands of the amplified product. The product is first bound to a solid support, for example polystyrene beads, either by hybridization to an oligonucleotide complementary to the primer or through a ligand attached to the primer. If biotin is linked to the primer, for example, the product would be isolated with a solid support coated with avidin or streptavidin. Contaminants can then be washed away. The product can then be released from the support by cleavage of the primer. For this purpose a single stranded ribonuclease is generally preferred. The primer incorporated into the product, as well as excess copies of the primer used in the amplification reaction, remain bound. The released fragment can then be detected as a final step in a diagnostic assay or sequenced. If the product is released using a single stranded ribonuclease or base treatment it will have a free 5′-OH group and can be readily labeled with $^{32}P$ using polynucleotide kinase.

For use in clinical and research applications, a kit for performing the process of this invention can be prepared with suitable substrates and reagents. Such a kit would include one or more primers or polynucleotide substrates able to hybridize to a specific target nucleic acid sequence and containing a ribose residue that becomes incorporated into the amplified product, and a chemical or enzymatic reagent used to cleave the ribonucleotide linkage to interfere with further replication of the product. If an enzyme is used as the cleaving reagent, its specificity must match the RNA sequence within the primer. For example, RNase$T_1$ could be used in conjunction with a primer containing a 3′-terminal ribo G residue. For alkaline hydrolysis of the ribonucleotide linkage a sodium or potassium hydroxide solution is preferred, and a reagent to neutralize the reaction mixture (see Example 3) can also be provided with the kit.

The following examples are offered to further illustrate, but not limit, the process, products and techniques of the invention.

## EXAMPLE 1

Use of RNA Containing Primers in PCR

The following example demonstrates the use of primers containing ribose residues in PCR. The target was human cytomegalovirus (CMV) DNA. A segment within this molecule of 130 residues was amplified. The primers used in the reaction were:

5'GCAGAGCTCGTTTAGTGAA(rC)

5'GGCACGGGGAATCCGCGTT(rC)

Both contain a terminal ribo C residue. For comparison, reactions were carried out with primers having the same sequence composed entirely of DNA. Reactions were performed in 100 ul containing 67 mM Tris-HCl pH 8, 7 mM $(NH_4)_2SO_4$, 7 mM $MgCl_2$, 10 mM 2-mercaptoethanol and 170 ug/ml BSA. The concentration of primers used was 1 uM. Each deoxynucleotide triphosphate was present at 0.8 mM. The template DNA varied from $10^4$ to $10^8$ molecules. Reactions were catalyzed with 2.5 units of Taq DNA polymerase (Amersham). Before the reaction, each sample was overlaid with 100 ul of light mineral oil to prevent sample evaporation. PCRs were performed in an Ericomp Twin Block System thermocycler for 20 to 30 cycles using the following program: 2 min. at 94°C, 1.5 min. at 65°C and 1 min. at 72°C. After the reaction the product was separated on a 2.5% agarose gel and visualized with ethidium bromide.

The level of amplification of the 130 base pair CMV fragment achieved with the primers terminating in ribo C was identical to that with the all DNA primers. Primers terminating in ribo A, U or G complementary to the same region of the CMV genome were next synthesized. Each served as an effective substrate for PCR. These studies establish that primers terminating in any of the four ribose residues can be extended with Taq DNA polymerase, and that the RNA residue once incorporated into the product can be copied in subsequent rounds of synthesis. Use of primers incorporating ribose residues required no modification of the PCR protocol.

## Example 2

Cleavage of Primers Containing a Ribose Residue from an Amplified Nucleic Acid Sequence

The 130 base pair CMV fragment synthesized in Example 1 with primers terminating in ribo C was isolated by electrophoresis on an 8% non-denaturing polyacrylamide gel:

```
5'————rC-----------------------------------------
  -----------------------------------------rC————5'
```

Both strands were labeled at their 5'-end with $^{32}P$ using T4 polynucleotide kinase. The product was then subjected to alkaline hydrolysis in 0.6 N NaOH at 90°C. Aliquots were taken from the reaction mixture at various times and the reaction was stopped by the addition of HCl to neutralize the solution. The products were analyzed by electrophoresis on a 10% denaturing polyacrylamide gel in the presence of 7 M urea. The gel was run for 2 hours at 600 V and then autoradiographed. After 30 minutes the starting material was no longer detectable. All of the radiolabel migrated in a single band corresponding to the cleaved primers.

Cleavage of the primers from the 130-mer was also accomplished with RNaseA. The product was dissolved in 0.01 M Tris pH 8 and heated at 90°C for 5 minutes to separate the two strands. The solution was then cooled to 37°C and 2 units of RNaseA (Boehringer Mannheim) were added. Complete cleavage of the primers was obtained within 30 minutes.

## Example 3

Protection Against Carry-over of Amplified Nucleic Acid Sequences in PCR Using RNA Containing Primers

This example demonstrates the use of RNA containing primers to eliminate the problem of contamination by amplified nucleic acid sequences in PCR. Samples containing 8.4 X $10^6$ copies of CMV DNA or the 130

base pair CMV fragment amplified with ribo C primers were dissolved in 10 ul of 0.6 N NaOH. The mixtures were heated to 90°C for 30 minutes and then neutralized by addition of 6 ul of 1 N HCl. PCR sample buffer (84 ul) was then added to give a final volume of 100 ul containing 60 mM NaCl, 67 mM Tris-HCl pH 8.8, 10 mM 2-mercaptoethanol, 7 mM $MgCl_2$, 170 ug/ml BSA, 0.25 uM of each primer and 0.2 mM of each deoxynucleotide triphosphate. After addition of 2.5 units of Taq DNA polymerase, the samples were overlaid with 100 ul of light mineral oil and 23 cycles of PCR were performed as described in Example 1. The products were analyzed by electrophoresis on a 2.5% agarose gel in the presence of ethidium bromide. The results are shown in Fig. 4. Lane 1 is the 130 base pair CMV fragment run as a standard. Lane 2 shows the amplified product after 23 cycles of PCR starting with 8.4 X $10^6$ molecules of the 130-mer which had not been base treated. With base treatment (lane 3), the product was not detectable. Lanes 4 and 5 show the results obtained with an equivalent amount of CMV DNA used as the substrate with and without base treatment, respectively. Base treatment did not interfere with amplification of the native target sequence. In fact, the level of amplification was somewhat increased, probably due to more effective denaturation of the DNA by the initial exposure to alkaline conditions at elevated temperatures. Similarly, base treatment did not interfere with amplification of the 130 base pair fragment produced with all DNA primers (not shown). Lane 6 is the no target control showing that the reaction was dependent on input of template.

These experiments demonstrate that RNA containing primers can be used in nucleic acid amplification reactions and that cleavage of the primers from an amplified sequence by selective hydrolysis of the ribonucleotide linkage prevents further replication of the product. It can further be seen that the invention accomplishes at least all of the objectives heretofore stated.

In summary, the present invention provides an improved method for eliminating the contamination problem associated with nucleic acid amplification reactions wherein novel primers or polynucleotide substrates incorporating ribose residues are utilized which can be cleaved from the amplified product. Removal of the primers from the product by selective hydrolysis of the ribonucleotide linkage prevents it from being further amplified. Since the process does not involve any modification of the DNA synthesized by primer extension or DNA within the sample, it can be carried out either pre- or post-amplification, or at both steps. The method can be used in conjunction with primer dependent amplification reactions including, but not limited to, PCR and transcription based systems, and amplification processes in which a polynucleotide substrate becomes directly incorporated into the product such as the ligase chain reaction. The method also provides an efficient means for isolating an amplified nucleic acid sequence wherein the product is first bound onto a solid support and then released by cleavage of the primer.

Other modifications of the embodiments of the invention described above which are obvious to those of skill in molecular biology and related fields can be made without departing from the scope and spirit of the invention and are intended to be encompassed within the following claims.

## Claims

**1.** A process for decreasing contamination by an amplified product in nucleic acid amplification reactions, said process comprising:

using a primer or polynucleotide substrate which contains a ribose residue in the amplification reaction; and

cleaving the ribonucleotide linkage within the amplified product.

**2.** A process as claimed in claim 1, in which cleavage of the ribonucleotide linkage is performed prior to the amplification reaction.

**3.** A process as claimed in claim 1, in which cleavage of the ribonucleotide linkage is performed after the amplification reaction.

**4.** A process as claimed in claim 1, 2 or 3, in which said amplification reaction is the polymerase chain reaction (PCR).

**5.** A process as claimed in claim 1, 2 or 3, in which said amplification reaction is a transcription based amplification reaction.

**6.** A process as claimed in claim 1, 2 or 3, in which said amplification reaction is the ligase chain reaction.

7. A process as claimed in any preceding claim, in which said primer contains a single ribose residue at its 3′-terminus.

8. A process as claimed in any preceding claim, in which cleavage of the ribonucleotide linkage is accomplished using an enzyme.

9. A process as claimed in claim 8, wherein said enzyme is selected from RNaseIII, RNaseH and single stranded ribonucleases.

10. A process as claimed in any one of claims 1 to 9, in which cleavage of the ribonucleotide linkage is accomplished by treatment with base.

11. A process for isolating an amplified nucleic acid sequence, said process comprising:
using a primer or polynucleotide substrate containing a ribose residue in the amplification reaction to produce said sequence;
binding said sequence to a solid support, and
releasing said sequence from said solid support by cleaving the ribonucleotide linkage.

12. A process as claimed in claim 11, in which said sequence is bound to a solid support through a biotin-avidin linkage.

13. A process as claimed in claim 11 or 12, in which the ribonucleotide linkage is cleaved using an enzyme.

14. A process as claimed in claim 13, in which said enzyme is a single stranded ribonuclease.

15. A process as claimed in claim 11 or 12, in which the ribonucleotide linkage is cleaved using base.

16. A kit for detection of a nucleic acid sequences which minimizes contamination by an amplified product comprising:
a primer or polynucleotide substrate able to hybridize to a specific target nucleic acid sequence and containing a ribose residue that becomes incorporated into an amplification product; and
a reagent able to cleave the ribonucleotide linkage of said primer or polynucleotide substrate.

17. A kit as claimed in claim 16, in which said primer contains a 3′ terminal ribonucleotide residue.

18. A kit as claimed in claim 16 or 17, in which said cleavage reagent is an enzyme.

19. A kit as claimed in claim 16 or 17, in which said cleavage reagent is a basic solution.

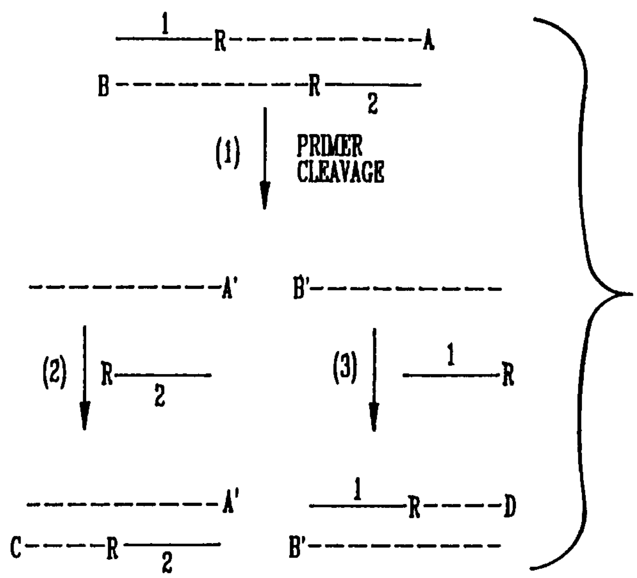
1
R
A
B
R
2
(1)
PRIMER
CLEAVAGE
A'
B'
(2)
R
2
(3)
1
R
A'
C
R
2
1
R
D
B'

FIG. 1

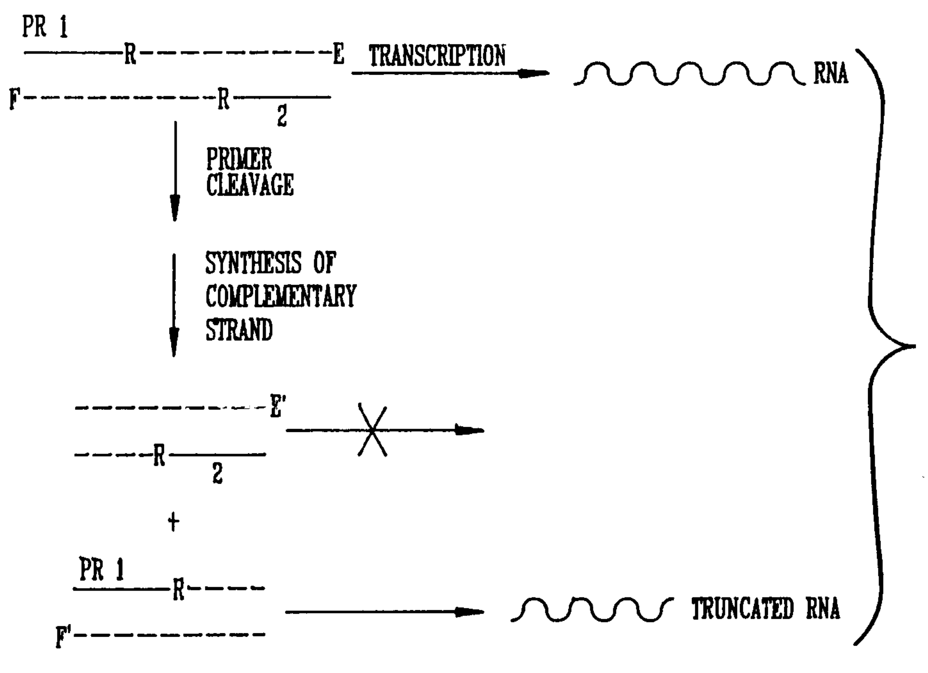
PR 1
R
E
TRANSCRIPTION
RNA
F
R
2
PRIMER
CLEAVAGE
SYNTHESIS OF
COMPLEMENTARY
STRAND
E'
R
2
+
PR 1
R
TRUNCATED RNA
F'

FIG. 2

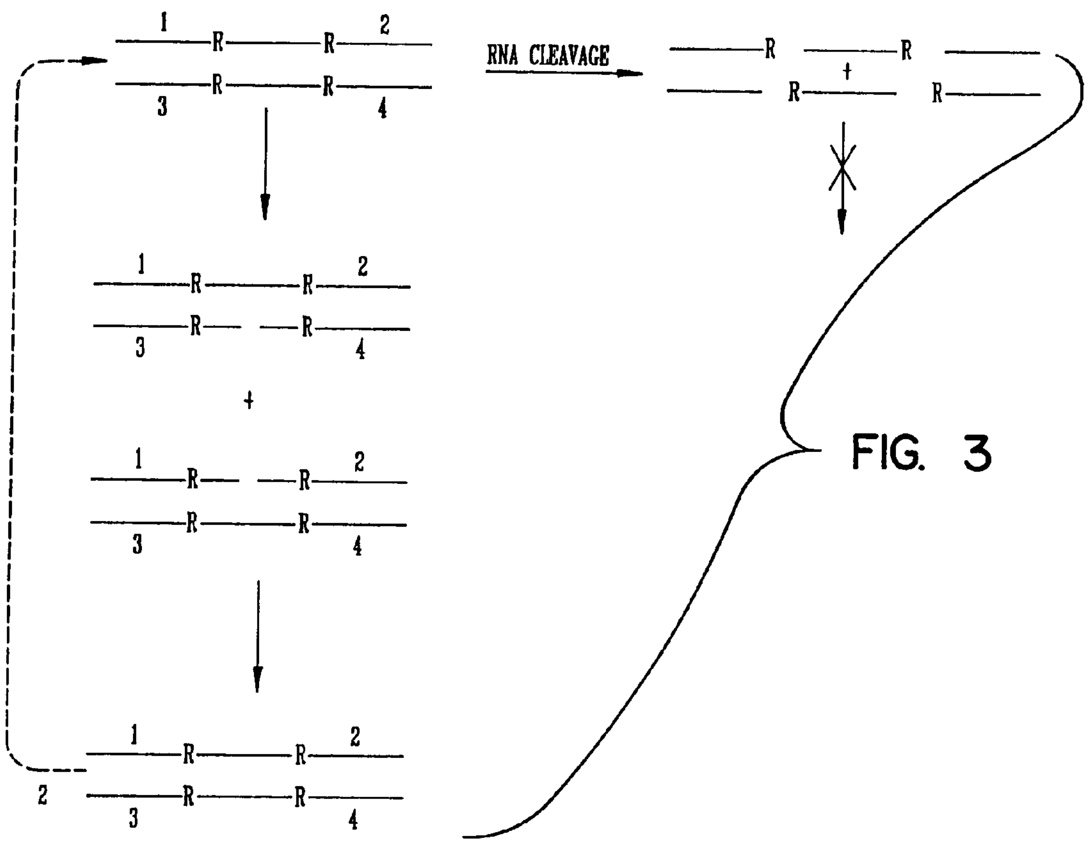


FIG. 3

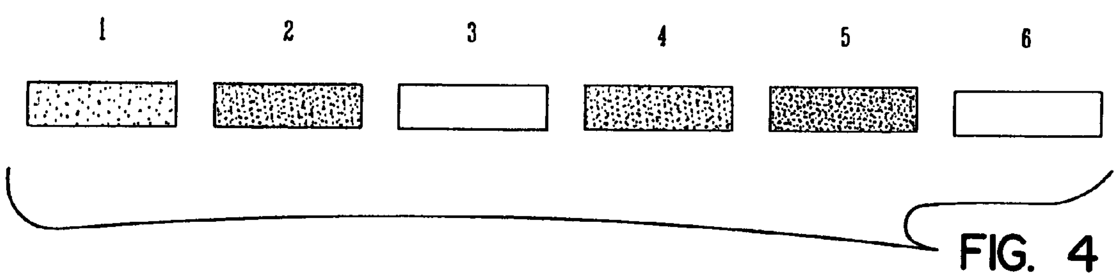


FIG. 4